# EUROPÄISCHE PATENTSCHRIFT

(11) **EP 2 577 573 B1**
(45) Veröffentlichungstag und Bekanntmachung des Hinweises auf die Patenterteilung: **10.02.2016**
(21) Anmeldenummer: 11722079.8
(22) Anmeldetag: 31.05.2011
(51) Int. Cl.: G06K 19/077, A61M 5/00, A61J 1/00, A61J 7/00, B01L 3/14, B01L 3/00, B65D 23/14, G01N 35/00, G06F 19/00

(54) **SICHERHEITSSYSTEM MIT EINEM TRANSPONDER UND EINEM LESEGERÄT EINES RFID-SYSTEMS**
SAFETY SYSTEM WITH A TRANSPONDER AND A READER FOR AN RFID SYSTEM
SYSTÈME DE SÉCURITÉ DOTÉ D'UN TRANSPONDEUR ET D'UN APPAREIL DE LECTURE D'UN SYSTÈME RFID

(30) Priorität: 07.06.2010 EP 10165127
(43) Veröffentlichungstag der Anmeldung: 10.04.2013
(73) Patentinhaber: Boehringer Ingelheim International GmbH, 55216 Ingelheim am Rhein (DE)
(72) Erfinder: BALTHES, Eduard, 55216 Ingelheim am Rhein (DE)
(74) Vertreter: Simon, Elke Anna Maria
(86) Internationale Anmeldenummer: PCT/EP2011/058871
(87) Internationale Veröffentlichungsnummer: WO 2011/154276

(56) Entgegenhaltungen:
- WO-A1-2010/000843
- WO-A2-2009/000425
- DE-A1-102004 046 003
- DE-A1-102005 052 122
- DE-A1-102007 034 155
- DE-A1-102007 055 635
- DE-U1-202007 014 281
- US-A1- 2002 013 614
- US-A1- 2002 188 259
- US-A1- 2003 011 476
- US-A1- 2004 050 860
- US-A1- 2005 162 277
- US-A1- 2007 150 032
- US-A1- 2008 197 042
- US-A1- 2009 009 290
- US-B1- 6 259 654
- US-B1- 6 335 907
- US-B1- 6 475 443

## Beschreibung

Die Erfindung bezieht sich auf ein Sicherheitssystem mit einem Transponder und einem Lesegerät eines RFID-Systems, wobei der Transponder einem Arzneimittel und das Lesegerät einer Applikationsvorrichtung für das Arzneimittel zugeordnet ist und die Applikationsvorrichtung nur nach einer Freigabe durch das RFID-System verwendbar ist.

Arzneimittel werden in den verschiedensten Darreichungsformen konfektioniert, um beispielsweise einen Wirkstoff zu spritzen, ein Aerosol einzuatmen oder um einen Wirkstoff über die Haut aufzunehmen. Häufig werden zur Einnahme und/oder Anwendung von Arzneimitteln zusätzliche Applikationsvorrichtungen benötigt, wie beispielsweise ein Inhalator, eine Spritze, ein Vernebler zur Erzeugung von Aerosolen oder dergleichen.

Die korrekte Zuordnung eines anzuwendenden Arzneimittels zu einer Applikationsvorrichtung, mit deren Hilfe das Arzneimittel angewendet oder eingenommen wird, ist somit von erheblicher Bedeutung, um eine falsch dosierte Anwendung von Arzneimitteln, Medikamenten bzw. Wirkstoffen oder die Anwendung eines gänzlich falschen Arzneimittels zu unterbinden.

Hierzu ist es unter anderem bekannt, bestimmte Applikationsvorrichtungen, insbesondere Inhalationsgeräte, die mit einer Kartusche, einer Flasche oder einem sonstigen mit einem Arzneimittel gefüllten Behälter zusammenwirken, derart auszubilden, dass beispielsweise nur eine spezifische Verpackungsform in eine Aufnahmeöffnung einsetzbar bzw. verrastbar ist. Somit können Arzneimittel eines anderen Arzneimittelherstellers nicht mit einer solchen Applikationsvorrichtung verwendet werden. Weiterhin können Farbkodierungen auf Arzneimittelverpackungen sowie den dazugehörigen Applikationsvorrichtungen vorgesehen sein, es können Warnhinweise auf Verpackungen sowie den Beipackzetteln enthalten sein oder die Anwendung besonders kritischer Arzneimittel ist nur dem medizinischen Fachpersonal vorbehalten.

Weiterhin sind Sicherheitssysteme zur Vermeidung einer falschen Anwendung eines Arzneimittels vorgeschlagen worden, bei denen einem Arzneimittel ein Transponder eines RFID-Systems zugeordnet ist. Bei RFID (englisch: "Radio frequency identification device")-Systemen handelt es sich um Systeme aus einem üblicherweise passiven Transponder und einem zugeordneten Lesegerät, um Gegenstände oder Personen mit Hilfe von elektromagnetischen Wellen zu identifizieren und/oder zu lokalisieren. Dabei ist der Transponder beispielsweise an einer Sekundärverpackung, wie einer Pappschachtel zur Aufnahme eines in einer Primärverpackung aufgenommenen Arzneimittels, angeordnet und kann in den Erfassungsbereich eines Lesegeräts an einer Applikationsvorrichtung gebracht werden. Das Lesegerät erkennt selbsttätig, dass ein zulässiger Transponder, der über die entsprechende Kodierung für dieses Arzneimittel verfügt, sich im Erfassungsbereich befindet. Nachfolgend kann von dem Lesegerät ein Freigabesignal ausgegeben werden, um die Benutzung der Applikationsvorrichtung zum Zusammenwirken mit dem Arzneimittel freizugeben.

US 2009/0009290 betrifft ein Dialysegerät mit einem RFID-System, welches Informationen hinsichtlich der Position eines medizinischen Anschlussstücks überprüft.

DE 10 2007 055 635 betrifft ein System zum Verabreichen eines Medikaments in flüssiger Form. Das System umfasst ein Medikamenten-Reservoir, eine Medikamenten-Dosiervorrichtung zum Verabreichen des flüssigen Medikaments und ein Medikamenten-Vorratsbehälter sowie eine Befüllvorrichtung zum automatischen Befüllen des Reservoirs mit dem Medikament aus dem Vorratsbehälter. Gemäss einer Ausführungsform weist die Befüllvorrichtung eine Erkennungsvorrichtung für eine automatische Erkennung des Medikaments aus dem Vorratsbehälter auf. So kann der Vorratsbehälter mit einem RFID versehen sein, welches über ein Lesegerät durch die Befüllvorrichtung erkannt wird.

US 6,259,654 offenbart ein Medikationsbehälter, welcher mehrere Behältnisse für unterschiedliche Medikationen organisiert. Ein Informationsstreifen ist an jedem Behältnis angebracht und enthält Informationen hinsichtlich der Dosierung.

DE 10 2004 046 003 offenbart ein pharmazeutisches Packmittel in Form einer Spritze mit einem unzugänglich oder integrierten Identifikationsmittel.

US 2002/0188259 bezieht sich auf Produktauthentifizierung mittels Identifikation und Zertifizierung der Herkunft oder des Herstellers von medizinischem Verbrauchsgut, Zubehör oder Geräten. US 2002/018859 offenbart ein medizintechnisches Gerät mit einem RFID-Lese-/Schreibgerät, das mit einer Serie von RFIDs gekoppelt ist.

DE 10 2007 034 155 bezieht sich auf eine Vorrichtung mit mindestens einer Verpackungseinheit mit einem zur Radiofrequenz-Identifikation dienenden RFID-Chip, der einen Schaltkreis sowie eine Antennenstruktur aufweist, und einer Sende-/Empfangseinheit zum Senden eines Sendesignals und/oder Empfangen eines Antwortsignals von dem RFID-Chip. Das Antwortsignal ist mittels einer an die Sende-/Empfangseinheit angeschlossenen Auswerteeinheit auswertbar. Der RFID-Chip weist eine Arbeitsfrequenz auf, die in einem Frequenzband unterhalb eines Bereichs einer Sperrfrequenz liegt, die durch die Leitfähigkeit der metallischen Aussenhülle und deren Dicke bestimmt ist.

US 2008/0197042 bezieht sich auf eine Blisterpackung mit Radiofrequenz-Identifikationseinrichtung, welche einen Trägerkörper aus flächigem Kunststoffmaterial, eine Aluminiumverschlussfolie, welche mit dem ebenen Bereich des Trägerkörpers flächig verbunden ist, und eine Radiofrequenz-Identifikationseinrichtung, welche in dem über die Aluminiumverschlussfolie hinausragenden Bereich des Trägerkörpers angebracht ist, umfasst.

US 2005/0162277 betrifft ein Produktsicherheitssystem mit einem RFID-Chip und einer Antenne, die an trennbare Teile eines handelsüblichen Produktpakets polymerisiert werden. Der RFID-Chip enthält eine eindeutige Seriennummer, die durch einen drahtlosen Leser abgefragt werden kann. Eine Datenbank mit solchen eindeutigen Seriennummern kann verwendet werden, um Fälschungen zu erkennen. Der RFID-Chip und die Antenne sind so eingebettet, dass Versuche diese zu entfernen oder zu übertragen, bei Überprüfung des Produktpaktes offensichtlich sind.

WO2010/000843 offenbart ein Sicherheitssystem zur Vermeidung einer falschen Arzneimittelanwendung eines Arzneimittels mit Hilfe einer Applikationsvorrichtung bestehend aus einem RFID-System aus Lesegerät und Transponder, die der Applikationsvorrichtung und dem Arzneimittel wechselseitig zugeordnet werden. Eine Verwendung der Applikationsvorrichtung ist nur dann möglich, wenn vom RFID-System eine Freigabe erteilt wird.

US 6,475,443 bezieht sich auf eine Vorrichtung, die ein Glasgehäuse mit einem Aufnahmeraum zum Aufbewahren von Chemikalien beinhaltet. Die Vorrichtung umfasst zudem einen Transponder, der derart in der Vorrichtung angeordnet ist, so dass er von den Chemikalien nicht beeinträchtigt wird.

Dabei ist es allerdings nicht auszuschließen, dass eine Manipulation dadurch erfolgt, dass das in einer Primärverpackung, wie einer Blisterfolie oder Kartusche, verpackte Arzneimittel von der Sekundärverpackung mit dem Transponder getrennt und beispielsweise ein gefälschtes Arzneimittel in die Sekundärverpackung eingefüllt wird. Auch können bei einem Anwender verschiedene Arzneimittel aus unterschiedlichen Verpackungen entnommen und falsch zurückverpackt werden. Auch ist die Produktfälschung nicht auszuschließen, wenn die Möglichkeit besteht, den Transponder von dem Arzneimittel bzw. dessen Verpackung zu trennen und einem anderen, beispielsweise gefälschten, Arzneimittel zuzuordnen.

Der Erfindung liegt die Aufgabe zugrunde, ein Sicherheitssystem der eingangs genannten Art zu schaffen, mit dem die Manipulationssicherheit, insbesondere die Sicherheit eines Patienten vor einem gefälschten Arzneimittel, erhöht und gleichzeitig eine falsche Anwendung des Arzneimittels wirkungsvoll unterbunden ist.

Die Erfindung ist durch Anspruch 1 definiert, der gegen die oben genannte WO 2010/000843 abgegrenzt wurde. Bevorzugte Ausführungsbeispiele gehen aus den abhängigen Ansprüchen hervor.

Bei einem derart ausgestalteten Sicherheitssystem ist der Transponder eines im Stand der Technik bekannten RFID-Systems beispielsweise innerhalb des Behälters oder einer Wandung des Behälters für das Arzneimittel untergebracht. Insbesondere ist wichtig, dass der Behälter nach seiner Befüllung mit dem Arzneimittel herstellerseitig verschlossen wird und der Transponder anschließend unzugänglich untergebracht ist.

Das bedeutet, dass beispielsweise bei der Verpackung des Arzneimittels in einer Blisterpackung, beim Einfüllen von Inhalationspulver oder Inhalationsflüssigkeiten in einen Ein- oder Mehrdosisinhalator, beim Einfüllen eines Arzneimittels in einen Behälter, insbesondere in Form einer Kapsel oder Kartusche, in eine Pen-Dosiereinrichtung, beim Konfektionieren eines Arzneimittels für einen Vernebler oder beim Befüllen einer Infusionseinrichtung in den Behälter, beispielsweise eine Primärverpackung, der Transponder in den entsprechenden Behälter mit eingebracht oder in Material zur Fertigung des Behälters eingebettet wird.

Selbstverständlich ist der Transponder mit einer entsprechenden Kodierung zur genauen Bezeichnung und/oder Anwendung des Arzneimittels versehen, um über das Lesegerät eine an sich beliebig ausgestaltbare Applikationsvorrichtung nur dann frei zu schalten, wenn das richtige Arzneimittel der Applikationsvorrichtung zugeführt wird. Dabei ist es im Rahmen der Erfindung mit umfasst, dass das Lesegerät dem Behälter unzugänglich zugeordnet und der Transponder an der Applikationsvorrichtung angeordnet ist. Auch kann es sich bei dem Transponder sowohl um einen passiven Transponder handeln, der durch elektromagnetische Wellen des Lesegeräts aktiviert beziehungsweise ausgelesen wird, als auch um einen aktiven Transponder, der selbstätig elektromagnetische Wellen ausstrahlt und hierzu mit einer Energiequelle, wie einer Batterie, ausgestattet ist. In gleicher Weise kann der Transponder auch in das Material des Behälters eingearbeitet sein. Als Material für einen Behälter kommen beispielsweise Kunststoff-Metall-Verbundmaterialien oder Sandwich-Systeme zum Einsatz, zwischen deren mehreren Lagen auch ein Transponder eingearbeitet sein kann. Entsprechende Werkstoffe für elastische oder starre Behälter können vom Fachmann in gewünschter Wandstärke verarbeitet werden.

Der Vorteil der Erfindung besteht darin, dass eine Manipulation des Transponders innerhalb des Behälters nur dann möglich ist, wenn dieser geöffnet beziehungsweise zerstört wird, wobei der Behälter vorzugsweise derart ausgelegt ist, dass ein Öffnen oder eine Manipulation unmittelbar seitens eines Benutzers feststellbar ist und nicht mehr rückgängig gemacht werden kann, damit der Benutzer oder Patient auch einen Rückschluss auf ein gefälschtes Arzneimittel beziehungsweise einen nicht mehr im Originalzustand befindlichen Behälter ziehen kann.

In Ausgestaltung umfasst der Behälter einen Innenbehälter, insbesondere einen elastischen Folienbehälter, für das Arzneimittel und einen mit dem Innenbehälter fest verbundenen Außenbehälter, insbesondere eine Kartusche, wobei der Transponder in einem zwischen dem Innenbehälter und dem Außenbehälter vorhandenen Zwischenraum angeordnet ist. Im einfachsten Fall wird der Transponder lose in dem Zwischenraum des doppelwandigen Behälters verstaut, bevor dieser mit dem darin aufgenommenen Arzneimittel verschlossen wird. Selbstverständlich ist auch ein doppelwandiger Beutel zur Aufnahme einer Wirkstoffformulierung denkbar.

Zweckmäßigerweise ist der Transponder an der Außenwandung des Innenbehälters oder der Innenwandung des Außenbehälters, vorzugsweise unverlierbar, befestigt. Beispielsweise wird der Transponder festgeklebt. Die Positionierung des Transponders an dem Behälter vereinfacht eine Signalübertragung zwischen dem Transponder und dem Lesegerät, wenn sich der Behälter zur bestimmungsgemäßen Verabreichung des Arzneimittels in der Applikationsvorrichtung befindet.

In alternativer Ausgestaltung umfasst der Behälter ein Laminat, zwischen dessen Schichten der Transponder angeordnet ist. Zweckmäßigerweise umfasst das Laminat mehrere Schichten aus mindestens einer Metall-Folie, insbesondere Aluminium-Folie, und mindestens einer Kunststoff-Folie, wobei die Metall-Folie eine Außenseite des Behälters, insbesondere einer Blister-Packung oder eines Beutels, bildet.

Vorzugsweise findet ein derartiges Sicherheitssystem Anwendung bei Primärverpackungen eines Arzneimittels, die zumindest teilweise metallische Komponenten enthalten. Dies sind beispielsweise Blisterpackungen mit einer Aluminium- oder Aluminium-Verbundfolie oder Behälter mit einem metallischen Deckel oder Siegel.

Aufgrund der bei einem RFID-Stystem verwendeten elektromagnetischen Wellen würde ein metallischer oder metallhaltiger Behälter erwartungsgemäß das Eindringen der elektromagnetischen Wellen vom Lesegerät außerhalb nach Innen zum Transponder hin unterbinden beziehungsweise abschirmen. Wird jedoch ein niederfrequentes RFID-System eingesetzt, können die niederfrequenten, langwelligen elektromagnetischen Wellen eine Wandung eines solchen Behälters zumindest gedämpft durchdringen und die Kodierung des Transponders kann ausgelesen werden. Dies hat sich insbesondere für Frequenzen von weniger als 500 kHz, vorzugsweise weniger als 30 kHz gezeigt. Derartige Frequenzen können auch metallische Schichten oder Folien einer Wandstärke größer als 0,3 bis 0,5 mm durchdringen, was üblichen Folienmaterialien bei Arzneimittelverpackungen entspricht.

Vorzugsweise kann insbesondere in der Kodierung im Transponder ein Verfallsdatum beziehungsweise ein Mindesthaltbarkeitsdatum des Arzneimittels hinterlegt sein, so dass nach dem dort hinterlegten Datum eine Verwendung des dann nicht mehr medizinisch sicheren Arzneimittels ausgeschlossen ist, da die Applikationsvorrichtung nicht mehr frei geschaltet wird.

Ebenso kann eine Individualisierung vorgenommen werden, um das Sicherheitssystem kindersicher zu machen. Hierzu kann einem befugten Nutzer ein als Freischalteinheit ausgelegtes Lesegerät, zum Beispiel in Form eines Armbandes oder einer Schmuckkette, einer SOS-Kapsel oder unter Umständen auch als Implantat zugeordnet werden. Somit können verschiedene medizinische Produkte nur dann freigegeben werden, wenn der Patient über eine entsprechende Freischalteinheit verfügt. Dabei kann das Sicherheitssystem derart ausgelegt werden, dass zum einen das Signal des Transponders von dem Lesegerät an der Applikationsvorrichtung ausgelesen wird und zum anderen ein Signal der Freischalteinheit angefordert und überprüft wird. Nur wenn beide Signale zulässig sind, kann das Medikament verwendet werden. Gleiches gilt beispielsweise für die Anwendung von Medikamenten auf Geriatriestationen, um bestimmten Patienten nur bestimmte Medikamente und/oder Dosierungen zuzuordnen.

Weiterhin ist es möglich, innerhalb des Behälters, insbesondere in dessen Zwischenraum, dem Arzneimittel und dem Transponder einen Feuchtigkeitsdetektor zuzuordnen, der bei Eindringen von Feuchtigkeit und der damit einhergehenden Gefahr einer negativen Beeinträchtigung des Arzneimittels oder seiner Brauch- bzw. Verabreichbarkeit anspricht und derart mit dem Transponder in Wirkverbindung steht, dass bei auftretender unerwünschter Feuchtigkeit keine Freischaltung der Applikationsvorrichtung erfolgt.

Schließlich können über den Transponder auch Datum und/oder Uhrzeit ausgelesen werden, um die Freigabe bestimmter Arzneimittel nur zu bestimmten Tageszeiten zu ermöglichen, wenn dies medizinisch sinnvoll ist.

Die Erfindung wird im Folgenden anhand von zwei Ausführungsbeispielen mit Bezugnahme auf die Zeichnung näher erläutert. Es zeigt:

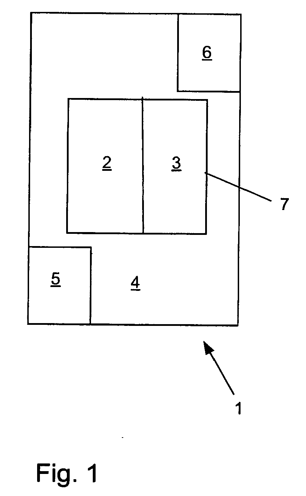

- Fig. 1: eine schematische Darstellung eines erfindungsgemäßen Sicherheitssystems und

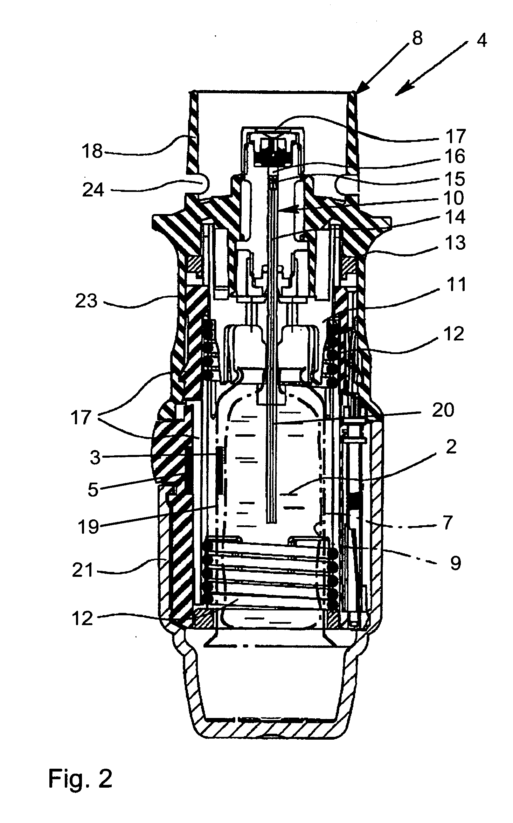

- Fig. 2: eine Schnittdarstellung des einen Zerstäuber umfassenden Sicherheitssystems nach Fig. 1.

Das Sicherheitssystem 1 dient dazu, die falsche Anwendung oder die Einnahme eines nicht identifizierten Arzneimittels 2 mittels einer Applikationsvorrichtung 4 zu vermeiden. Hierzu ist einem Behälter 7 für das Arzneimittel 2 ein Transponder 3 unzugänglich zugeordnet, vorzugsweise in Form eines entsprechenden Magnetstreifens. Des Weiteren ist der Applikationsvorrichtung 4 ein Lesegerät 5 zugeordnet. Der Behälter 7 mit dem Arzneimittel 2 muss zur Anwendung oder Einnahme des Arzneimittels 2 mit der Applikationsvorrichtung 4 zusammenwirken, beispielsweise ein in einer Flasche portioniertes flüssiges Medikament mit einer Injektionsvorrichtung.

Das Lesegerät 5 erkennt, ob der Transponder 3 mit einem korrekten Kode für das Arzneimittel 2 versehen ist, wobei die Applikationsvorrichtung 4 mit verschiedenen Arzneimitteln 2 mit jeweils unterschiedlichen Transpondern 3 zusammenwirken kann. Wird der dem Behälter 7 und damit dem Arzneimittel 2 zugeordnete Transponder 3 vom Lesegerät 5 als korrekt bewertet, wird die Applikationsvorrichtung 4 mechanisch und/oder elektronisch frei geschaltet, um das Arzneimittel 2 einnehmen oder anwenden zu können. Gleichzeitig kann über eine Anzeigevorrichtung 6, beispielsweise einen Lautsprecher und/oder eine LED, ein entsprechendes Bestätigungs- oder Ablehnungssignal im Falle eines falschen Arzneimittels 2 bzw. Behälters 7 ausgegeben werden.

Der Behälter 7 umfasst metallische Bestandteile. Dabei hat es sich gezeigt, dass insbesondere bei niederfrequenten RFID-Systemen mit Frequenzen, bevorzugt kleiner oder gleich 500kHz, vorzugsweise kleiner oder gleich 30 kHz, die vom Lesegerät 5 ausgehenden elektromagnetischen Wellen den metallischen oder metallhaltigen Behälter 7 durchdringen und mit dem Transponder 3 zusammenwirken können, um die Applikationsvorrichtung 4 freizuschalten. Normalerweise schirmt ein derartiger Behälter 7 die elektromagnetischen Wellen des Lesegeräts 5 ab, jedoch wird bei geeigneter Wahl der Frequenz sowie einem geeigneten metallischen Material für den Behälter 7, beispielsweise eine Metall-Kunststoff-Sandwichkonstruktion mit einer Dicke von 0,3 bis 0,5 mm, diese durchdrungen. Hierbei kann ein Magnetstreifen bzw. ein Transponderchip zwischen mehreren Lagen der Sandwichkonstruktion eingearbeitet sein.

Die als Zerstäuber 8 ausgebildete Applikationsvorrichtung 4 dient zur Zerstäubung des fluiden Arzneimittels 2 und ist als tragbarer Inhalator ausgebildet, der ohne Treibgas arbeitet. Bei der Zerstäubung des Arzneimittels 2, vorzugsweise einer Flüssigkeit, wird ein Aerosol gebildet, das von einem Benutzer eingeatmet werden kann.

Der Zerstäuber 8 weist den auswechselbaren Behälter 7 mit dem Arzneimittel 2 auf, wobei der doppelwandige Behälter 7 im Wesentlichen einen zylindrischen Aufbau aufweist und von unten in den geöffneten Zerstäuber 8 einsetzbar ist. In einem starren Außenbehälter 19 des Behälters 7, der aus einem Metall gefertigt ist, befindet sich ein das Arzneimittel 2 aufnehmender, als elastischer Folienbehälter ausgebildeter Innenbehälter 9. In dem zwischen dem Außenbehälter 19 und dem Innenbehälter 9 vorhandenen Zwischenraum, der nach dem Verschließen des mit dem Arzneimittel 2 gefüllten Behälters 7 nicht zugänglich ist, befindet sich der Transponder 3, der mit dem Lesegerät 5 in dem Zerstäuber 8 zusammenwirkt.

Zur Zerstäubung des Arzneimittels 2 in einer vorbestimmten einstellbaren Menge umfasst der Zerstäuber 8 einen einen Kolben 20 umfassenden Druckerzeuger 10 mit einer Halterung 11 für den Behälter 7, einer Antriebsfeder 12 mit einer zur Entspannung manuell zu betätigenden Lösetaste 13, einem Förderrohr 14 mit einem eingesetzten Rückschlagventil 15, einer Druckkammer 16 und einer Düse 17, der ein Mundstück 18 zugeordnet ist.

Beim axialen Spannen der Antriebsfeder 12 durch ein Drehen eines Gehäuseunterteils 21 mit einem daran lösbar befestigten Innenteil 22 relativ zu einem an dem Mundstück 18 angeformten Gehäuseoberteil 23 wird die Halterung 11 mit dem Behälter 7 und dem Förderrohr 14 nach unten bewegt und Fluid aus dem Behälter 7 über das Rückschlagventil 15 in die dem Kolben 20 des Druckerzeugers 10 zugeordnete Druckkammer 16 gesaugt. Beim anschließenden schlagartigen Entspannen der Antriebsfeder 17 durch die Betätigung der Lösetaste 13 wird das Arzneimittel 2 in der Druckkammer 16 von der das Förderrohr 14 nach oben verlagernden Antriebsfeder 12 unter Druck gesetzt und über die Düse 17 ausgegeben, wobei eine Zerstäubung stattfindet. Die Zerstäubung erfolgt beispielsweise in Partikel im µm-Bereich, vorzugsweise in Partikel mit einer Größe von etwa 20 µm, die eine Wolke bzw. einen Strahl eines Aerosols bilden. Ein Benutzer kann das Aerosol inhalieren, wobei Zuluft über Zuluftöffnungen 24 in dem Mundstück 18 ansaugbar ist.

Kann das Lesegerät 5 von dem Transponder 3 kein zulässiges Signal empfangen, dann ist die Lösetaste 13 über eine nicht dargestellte Vorrichtung zwangsweise gesperrt und es kann kein Arzneimittels 2 zerstäubt werden.

## Patentansprüche

1. Sicherheitssystem umfassend eine Applikationsvorrichtung (4), einen Behälter (7) für ein Arzneimittel, einen Transponder (3) und ein Lesegerät (5) eines RFID-Systems mit einer Frequenz ≤500 kHz,
wobei das Lesegerät (5) der Applikationsvorrichtung (4) für das Arzneimittel (2) zugeordnet ist,
die Applikationsvorrichtung (4) nur nach einer Freigabe durch das RFID-System verwendbar ist und
der Behälter (7) in die Applikationsvorrichtung (4) einsetzbar ist
**dadurch gekennzeichnet, dass** der Behälter (7) mindestens eine metallische Schicht umfasst und einen Innenbehälter (9) für das Arzneimittel (2) und einen mit dem Innenbehälter (9) fest verbundenen Aussenbehälter (19) umfasst, wobei der Innenbehälter (9) in Form eines elastischen Folienbehälters und der Aussenbehälter (19) in Form einer Kartusche ausgebildet ist und wobei der Transponder (3) in einem zwischen dem Innenbehälter (9) und dem Aussenbehälter (19) vorhandenen Zwischenraum angeordnet ist.

2. Sicherheitssystem nach Anspruch 1, **dadurch gekennzeichnet, dass** der Transponder (3) an der Aussenwandung des Innenbehälters (9) oder der Innenwandung des Aussenbehälters (19) befestigt ist.

3. Sicherheitssystem nach einem der Ansprüche 1 oder 2, **dadurch gekennzeichnet, dass** die metallische Schicht eine Wandstärke zwischen 0,3 und 0,5 mm aufweist.

4. Sicherheitssystem nach einem der Ansprüche 1 bis 3, **dadurch gekennzeichnet, dass** das RFID-System ein Niederfrequenz-RFID-System mit einer Frequenz ≤ 30 kHz ist.

5. Sicherheitssystem nach einem der Ansprüche 1 bis 4, **dadurch gekennzeichnet, dass** im Transponder (3) ein Verfalldatum des Arzneimittels (2) hinterlegt ist.

6. Sicherheitssystem nach einem der Ansprüche 1 bis 5, **dadurch gekennzeichnet, dass** dem Arzneimittel (2) und dem Transponder (3) ein Feuchtigkeitsdetektor zugeordnet ist.

7. Sicherheitssystem nach einem der Ansprüche 1 bis 6, **dadurch gekennzeichnet, dass** im Transponder (3) Benutzungsdaten und Benutzungsuhrzeiten des Arzneimittels (2) hinterlegt sind.

## Claims

1. Safety system comprising an application device (4), a container (7) for a medicament, a transponder (3) and a reading device (5) of an RFID system with a frequency of ≤ 500 kHz,
the reading device (5) being associated with the application device (4) for the medicament (2),
the application device (4) being usable only after being enabled by the RFID system, and
the container (7) being insertable in the application device (4),
**characterised in that** the container (7) comprises at least one metallic layer and has an inner container (9) for the medicament (2) and an outer container (19) fixedly attached to the inner container (9), the inner container (9) being in the form of an elastic foil container and the outer container (19) being in the form of a cartridge, and the transponder (3) being arranged in an intermediate space located between the inner container (9) and the outer container (19).

2. Safety system according to claim 1, **characterised in that** the transponder (3) is attached to the outer wall of the inner container (9) or to the inner wall of the outer container (19).

3. Safety system according to one of claims 1 or 2, **characterised in that** the metallic layer has a wall thickness of between 0.3 and 0.5 mm.

4. Safety system according to one of claims 1 to 3, **characterised in that** the RFID system is a low frequency RFID system with a frequency of ≤ 30 kHz.

5. Safety system according to one of claims 1 to 4, **characterised in that** an expiry date of the medicament (2) is stored in the transponder (3).

6. Safety system according to one of claims 1 to 5, **characterised in that** a moisture detector is associated with the medicament (2) and the transponder (3).

7. Safety system according to one of claims 1 to 6, **characterised in that** the dates and times of use of the medicament (2) are stored in the transponder (3).

## Revendications

1. Système de sécurité comprenant un dispositif d'application (4), un contenant (7) pour un médicament, un transpondeur (3) et un appareil de lecture (5) d'un système RFID présentant une fréquence ≤ 500 kHz,
l'appareil de lecture (5) étant associé au dispositif d'application (4) destiné au médicament (2),
le dispositif d'application (4) ne pouvant être utilisé qu'après une validation par le système RFID et
le contenant (7) pouvant être inséré dans le dispositif d'application (4),
**caractérisé en ce que** le contenant (7) comprend au moins une couche métallique et comprend un contenant intérieur (9) destiné au médicament (2) et un contenant extérieur (19) relié de manière solidaire au contenant intérieur (9), le contenant intérieur (9) étant réalisé sous la forme d'un contenant en film élastique et le contenant extérieur (19) étant réalisé sous la forme d'une cartouche, et le transpondeur (3) étant disposé dans un espace intermédiaire existant entre le contenant intérieur (9) et le contenant extérieur (19).

2. Système de sécurité selon la revendication 1, **caractérisé en ce que** le transpondeur (3) est fixé au niveau de la paroi extérieure du contenant intérieur (9) ou au niveau de la paroi intérieure du contenant extérieur (19).

3. Système de sécurité selon l'une quelconque des revendications 1 ou 2,
**caractérisé en ce que** la couche métallique présente une épaisseur de paroi comprise entre 0,3 et 0,5 mm.

4. Système de sécurité selon l'une quelconque des revendications 1 à 3, **caractérisé en ce que** le système RFID est un système RFID basse fréquence présentant une fréquence ≤ 30 kHz.

5. Système de sécurité selon l'une quelconque des revendications 1 à 4,
**caractérisé en ce qu'**une date d'expiration du médicament (2) est mémorisée dans le transpondeur (3).

6. Système de sécurité selon l'une quelconque des revendications 1 à 5,
**caractérisé en ce qu'**un détecteur d'humidité est associé au médicament (2) et au transpondeur (3).

7. Système de sécurité selon l'une quelconque des revendications 1 à 6,
**caractérisé en ce que** des données d'utilisation et des horaires d'utilisation du médicament (2) sont mémorisés dans le transpondeur (3).
